# EUROPEAN PATENT APPLICATION

(11) **EP 3 633 050 A2**
(43) Date of publication of application: **08.04.2020**
(21) Application number: 18759145.8
(22) Date of filing: 29.05.2018
(51) Int. Cl.: C12Q 1/6883, C12Q 1/6876

(54) **IN VITRO METHOD FOR DIAGNOSING ASTHMA OR SUBFORMS THEREOF**

(30) Priority: 29.05.2017 ES 201730739
(71) Applicant: Fundacion Instituto De Investigacion Sanitaria Fundacion Jimenez Diaz, 28040 Madrid (ES); Centro de Investigación Biomédica en Red (CIBER), 28029 Madrid (ES); Fundación para la Investigación Biomédica del Hospital Universitario de la Paz, 28046 Madrid (ES); Fundación Conchita Rábago de Jiménez Díaz, 28001 Madrid (ES)
(72) Inventor: DEL POZO ABEJÓN, Victoria, 28040 Madrid (ES); SASTRE DOMINGUEZ, Joaquín, 28040 Madrid (ES); RODRIGO-MUÑOZ, José Manuel, 28040 Madrid (ES); SASTRE TURRIÓN, Beatriz, 28029 Madrid (ES); QUIRCE GANCEDO, Santiago, 28046 Madrid (ES); CAÑAS MAÑAS, José Antonio, 28001 Madrid (ES)
(74) Representative: Hoffmann Eitle
(86) International application number: PCT/ES2018/070390
(87) International publication number: WO 2018/220249

(57) **Abstract**

The present invention shows that miR-185-5p has higher relative expression levels (2^{-ΔCt}; where ΔCt = Ct of the miRNA of interest (miR-185-5p) - Ct of the endogenous miRNA) in patients with asthma compared with healthy individuals. Furthermore, not only are the expression levels of miR-185-5p different between healthy individuals and individuals with asthma, but also between healthy individuals and each of the subgroups of individuals with (intermittent, mild persistent, moderate, and severe) asthma. The results furthermore show that the last two stages of severity, i.e., the moderate and severe persistent stages, show a progressive increase or escalation of the expression thereof compared with mild intermittent and persistent stages.

## Description

### Field of the invention

The present invention can be included in the field of personalized medicine in which specific biomarkers are used for identifying a given disease or disorder. Specifically, some microRNAs (also referred to as miRNAs or miR-) are used in the present invention for identifying human subjects with the risk of developing both allergic- and non-allergic-type bronchial asthma, as well as for identifying different stages of severity in patients with bronchial asthma.

### Background of the invention

microRNAs are one of the most promising biomarkers with increasing importance in clinical application. microRNAs (miRNAs) are non-coding RNA sequences which have the size of 19 to 22 nucleotides and regulate gene expression on the post-transcriptional level. miRNAs bind to mRNA by base complementarity, inhibiting the translation of recognized mRNAs. One and the same miRNA can regulate many genes by means of the complementary binding thereof to different 3' untranslated regions (UTRs) of mRNAs, where it has been estimated that thousands of human genes are the targets of different miRNAs. Several studies have shown the effect of miRNAs in different Th2-type pathologies, such as asthma, eosinophilic esophagitis, allergic rhinitis, and atopic dermatitis, where it has been observed that miRNAs regulate a number of genes involved in these pathologies: miR-21 and interleukin (IL)-12, miR-126 with IL-5 and IL-13, and Let-7 with IL-13.

miRNAs obtained from serum or plasma samples are highly resistant to the action of RNAses at extreme temperature and other extreme conditions, making them very promising biomarkers for different diseases such as cancer.

From the first study characterizing miR-21 as being able to used as a biomarker in B-cell lymphoma, the field of study of miRNAs as biomarkers has undergone exponential evolution in recent years.

In this sense, a study by Solberg, O. D. et al. Airway Epithelial miRNA Expression Is Altered in Asthma. Am. J. Respir. Crit. Care Med. 186, 965-974 (2012), shows that the miRNA expression profiles between patients with asthma and healthy controls vary in airway epithelium and in exosomes isolated from bronchoalveolar lavage (BAL). miRNA expression has also been characterized in lymphocytes of child patients with asthma compared with healthy individuals.

Other groups studied expression in the specific cells of healthy individuals and individuals with asthma, with differences in miR-140 being seen in smooth muscle cells, or miR-223 and miR-146b in macrophages, Jardim, M. J., Dailey, L., Silbajoris, R. & Diaz-Sanchez, D. Distinct MicroRNA Expression in Human Airway Cells of Asthmatic Donors Identifies a Novel Asthma-Associated Gene. Am. J. Respir. Cell Mol. Biol. 47, 536-542 (2012), isolated bronchial epithelial cells from individuals with asthma and health individuals, where differences in the expression of miR-203 affecting the aquaporin 4 gene (AQP4) are observed. On the other hand, it has been found that miR-21 and miR-126 have greater expression in patients with asthma, with correlation between the levels of these miRNAs and IL-13 in epithelial cell cultures. Suojalehto, H. et al. Altered MicroRNA Expression of Nasal Mucosa in Long-Term Asthma and Allergic Rhinitis. Int. Arch. Allergy Immunol. 163, 168-178 (2014), which used nasal biopsies, showed different miRNA profiles between patients with asthma (with and without allergic rhinitis) and healthy controls with positive correlation between miR-155 and the values of FE_{NO} (Fraction of Exhaled Nitric Oxide), N_{NO} (Nasal Nitric oxide), and IL-13.

On the other hand, a sputum study showed that miR-629-3p, miR-223-3p, and miR-142-3p are increased in individuals with severe neutrophilic asthma and correlated with the high neutrophil levels.

miRNA expression in patients with asthma, patients with COPD (chronic obstructive pulmonary disease), and healthy individuals was studied in an exhaled breath condensate, with differences and similarities being seen between both pathologies. Similarly, another group of researchers used exosomes from an exhaled breath condensate, identifying differences for 11 miRNAs between healthy individuals and individuals with asthma.

Kho, A. T. et al. Circulating MicroRNAs: Association with Lung Function in Asthma. PLoS One 11, e0157998 (2016), studied the serum miRNA expression of patients with mild and severe asthma, determining that some miRNAs show association with the FEV1 (Forced Expiratory Volume)/FVC (Forced Vital Capacity) value, the% of FEV1, and the% of FVC, and act on FEV1 and asthma genes. Panganiban, R. P. L. et al. Differential microRNA expression in asthma and the role of miR-1248 in regulation of IL-5. Am. J. Clin. Exp. Immunol. 1, 154-65 (2012), analyzed the serum miRNA expression of individuals with asthma and healthy controls, observing differences in the expression of miR-1248, miR-26a, Let7a, and Let7d, as well as a negative correlation between miR-26a and the FEV1 value. Furthermore, they determined that IL-5 is positively regulated by miR-1248.

On the other hand, miR-21 is a microRNA which has been studied as a biomarker in various pathologies, including asthma. The levels of miR-21 serve as a biomarker both for differentiating individuals with asthma from healthy individuals and for treatment with corticoids. Sawant, D. V et al. Serum MicroRNA-21 as a Biomarker for Allergic Inflammatory Disease in Children. MicroRNA (Shariqah, United Arab Emirates) 4, 36-40 (2015), also characterized a greater expression of miR-21 in the biopsies and serum of patients with asthma and eosinophilic esophagitis. Panganiban, R. P. L. et al. Differential microRNA expression in asthma and the role of miR-1248 in regulation of IL-5. Am. J. Clin. Exp. Immunol. 1, 154-65 (2012), showed differences in the plasma expression of patients with asthma, patients with allergic rhinitis, and healthy controls.

The functionality of various miRNAs was evaluated *in vivo* in other studies. It was demonstrated in mouse models that the inhibition of miR-126 inhibited eosinophil recruitment and that miR-19a acts by promoting Th2 cytokine synthesis in airways by means of miR-17-92 cluster knock out and overexpression studies.

On the other hand, miR-3162-3p has been studied in lung cell cultures in relation to its effects on β-catenin suppression in asthma. An increased level of this miRNA and a decreased level of β-catenin have been detected in asthma in mouse models, and a miRNA inhibitor has been found to reduce airway hyperreactivity. Furthermore, miR-221 is known to reduce the expression of kinases, p21^{kip1} and p21^{WAF1}, increasing smooth muscle cell proliferation. It was found in this same cell population that miR-10a suppressed the signaling pathway by means of PI3K affecting PIK3CA, thereby inhibiting cell proliferation, detecting in turn that miR-146a was capable of reducing the expression of COX-2 and IL-1β, and in turn also reducing the expression of HuR (RNA-binding human antigen R protein). This miRNA is also known to inhibit inflammation caused by IL-1β in alveolar epithelial cells.

According to the foregoing, differences in the expression of a variety of miRNAs have been observed between healthy individuals and individuals suffering asthma. However, there is still the need to provide clinical tools with higher sensitivity which, unlike the actions of other markers (eosinophils, exhaled nitric oxide, periostin), are capable of backing the diagnosis of asthma in a manner independent of the presence of inflammation exclusively mediated by Th2, given that not all the patients with asthma (about 50%) present an overexpression of this inflammation-causing pathway, and which furthermore allow helping to classify asthma severity into mild intermittent or persistent asthma and other more severe forms of the disease.

### Brief description of the invention

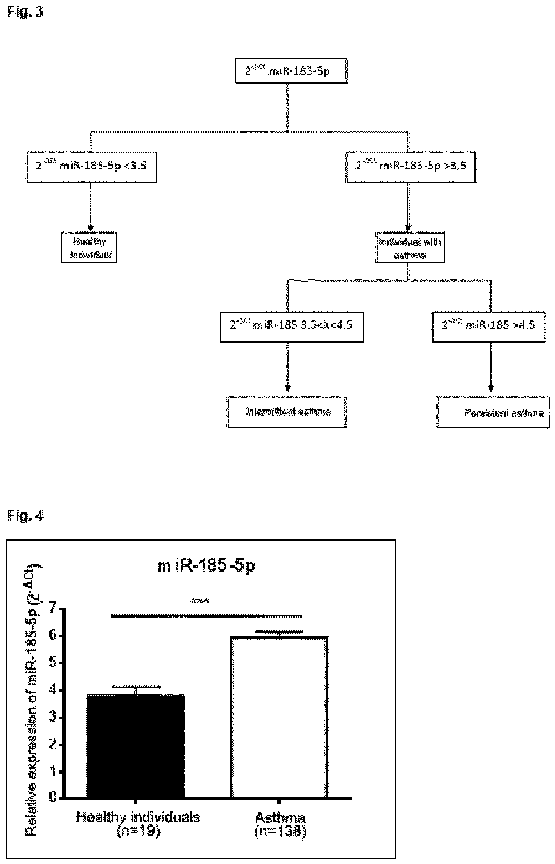

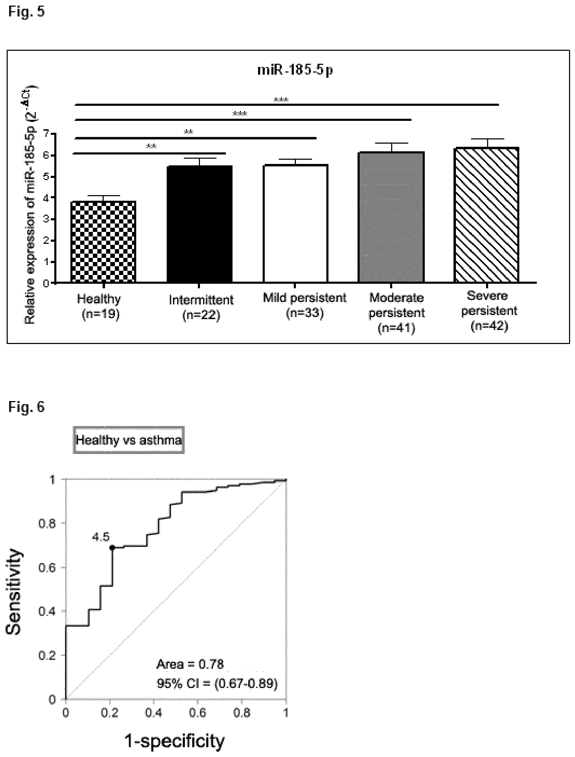

The present invention shows that miR-185-5p has higher relative expression levels (2^{-ΔCt}; where ΔCt = Ct of the miRNA of interest (miR-185-5p) - Ct of the endogenous miRNA) in patients with asthma compared with healthy individuals (see Figure 4). Furthermore, not only are the expression levels of miR-185-5p different between healthy individuals and individuals with asthma, but also between healthy individuals and each of the subgroups of individuals with (intermittent, mild persistent, moderate persistent, and severe persistent) asthma. The results furthermore show that, at least in the last two stages of severity, i.e., in moderate and severe persistent stages, the expression of miR-185-5p in serum samples shows a progressive increase or escalation compared with patients with asthma who show a mild intermittent asthma- or persistent asthma-related pathological feature (Figure 5).

Based on the foregoing data, it is understood that this biomarker, miR-185-5p, in samples obtained in a non-invasive manner from blood, serum, or plasma will be very useful in the diagnosis of patients with asthma regardless of the asthma endotype that is present. Furthermore, as can be seen throughout the present invention the biomarker, miR-185-5p, is capable of backing the diagnosis of asthma in a manner independent of the presence of inflammation exclusively mediated by Th2.

### Brief description of the Drawings

**Figure 1****.** Diagnosis algorithm of asthma.
**Figure 2****.** Current treatments for asthma and the stratification thereof for the different treatments according to the Spanish Guideline on the Management of Asthma (GEMA 4.0) in adults.
**Figure 3****.** Possible diagnosis algorithm to be used with miR-185-5p for clinical diagnosis.
**Figure 4****.** Relative expression of miR-185-5p between healthy individuals and individuals with asthma.
**Figure 5****.** Relative expression of miR-185-5p between healthy individuals and the different subgroups of individuals with asthma.
**Figure 6****.** Area under the curve/Sensitivity-specificity analysis of the relative expression of miR-185-5p between healthy individuals and individuals with asthma.
**Figure 7****.** Area under the curve/Sensitivity-specificity analysis of the relative expression of miR-185-5p between healthy individuals and the different subgroups of individuals with asthma according to severity.
**Figure 8****.** Relative expression of miR 185-5p between individuals with adolescent-onset and adult-onset asthma. The patients included in the study which brought about this drawing were asked about the age of asthma onset and the ages were collected (value from age 1 to age 99), being grouped into two groups according to the age of asthma onset: adolescent-onset asthma (from age 1 to age 17) and adult-onset asthma (from age 18 to age 99).
**Figure 9****.** This drawing shows how patients with asthma who have been admitted at least once to intensive care unit or ICU have higher expression levels of miR-185-5p than individuals with asthma.
**Figure 10****.** Logistic regression model in which the expression of miRNAs, miR-185-5p, miR-144-5p, and miR-1246 as a whole increase sensitivity and specificity of asthma diagnosis compared with the miRNAs individually.

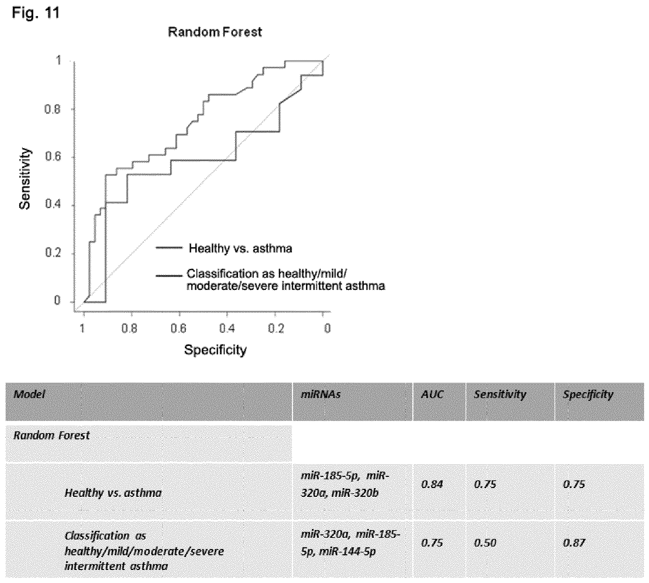

**Figure 11****.** Random Forest model (red line) in which the expression of the miRNAs, miR-185-5p, miR-320a, miR-320b as a whole increase sensitivity and specificity of asthma diagnosis compared with the miRNAs individually, whose capacity to perform diagnosis between healthy individuals and individuals with asthma is, however, similar to the logistic regression model. However, the second model (blue line) is capable of classifying an individual based on the expression of miR-320a, miR-185-5p, and miR-144-5p in healthy individual, individual with intermittent asthma, mild persistent asthma, moderate persistent asthma, or severe persistent asthma.
**Figure 12****:** miR-144-5p is overexpressed in patients with asthma compared with healthy individuals.
**Figure 13****.** Patients treated with anti-leukotrienes have higher relative expression levels of miR-144-5p than non-treated patients.
**Figure 14****.** miR-144-5p is overexpressed in patients with asthma who have been subjected to treatment with bronchodilators.
**Figure 15****.** miR-1246 is overexpressed in patients with asthma compared with healthy individuals.
**Figure 16****.** The expression levels of miR-1246 can be used as an asthma biomarker since its AUC is greater than 0.7.
**Figure 17****.** Patients treated with anti-leukotrienes have higher relative expression levels of miR-1246 than non-treated patients.
**Figure 18****.** miR-320a is overexpressed in patients with asthma compared with healthy individuals.
**Figure 19****.** The expression levels of miR-320a can be used as an asthma biomarker since its AUC is greater than 0.7.
**Figure 20****.** Patients treated with anti-leukotrienes have higher relative expression levels of miR-144-5p than non-treated patients.
**Figure 21****.** Relative expression levels of the miRNAs under study in healthy individuals and patients with asthma.

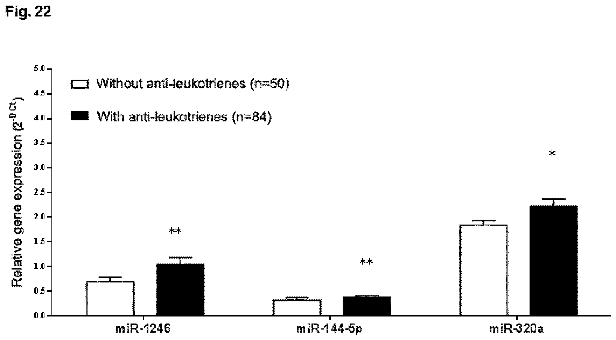

**Figure 22****.** Relative expression levels of differentially expressed miRNAs between patients with asthma treated with anti-leukotrienes and patients with asthma not treated with this medication.

### Detailed Description of the Invention

### Definitions

For the purposes of the present invention, the following definitions are included below:
- In the present invention, "asthma" is understood to be a chronic inflammatory disease of the respiratory tracts, whose pathogenesis involves various types of cells and inflammation mediators, is partly determined by genetic factors, and occurs with bronchial hyperresponsiveness (BHR) and varying degrees of airflow obstruction, completely or partially reversible, either spontaneously or as a result of pharmacological action.
- the term "selection" is understood to be the examination or test performed on a group of individuals belonging to the general population with the risk of suffering asthma for the purpose of distinguishing healthy individuals from those suffering asthma, more particularly for the purpose of distinguishing asthma severity between those individuals suffering asthma. In order to illustrate this selection, the present invention provides a diagnosis algorithm useful for setting the selection criteria according to the present definition, see Figure 3.
- In the present invention, the term "miR-185-5p" refers to the miRNA with the following mature sequence: 5'-UGGAGAGAAAGGCAGUUCCUGA-3'. As shown in the drawings of the present invention, the relative expression of miR-185-5p (compared with an endogenous miRNA similarly expressed in healthy individuals and individuals with asthma) is greater in patients with asthma when compared with the expression in healthy individuals. This greater expression in patients furthermore allows distinguishing healthy individuals from individuals with asthma due to the fact that the analysis of the area under the curve ROC is 0.78 with CI (95%) of 0.67-0.89, see Figure 6.
- The expression "positively regulated" or "overexpressed," referring to any of the microRNA or combinations thereof described in the present invention, refers to an increase in their expression level with respect to a given "threshold value" or "limit value" of at least 5%, at least 10%, at least 15%, at least 20%, at least 25%, at least 30%, at least 35%, at least 40%, at least 45%, at least 50%, at least 55%, at least more than 60%, at least more than 65%, at least 70%, at least 75%, at least 80%, at least 85%, at least 90%, at least 95%, at least 100%, at least 110%, at least 120%, at least 130%, at least 140%, at least 150%, or more.
- In the present specification, the Anglo-saxon punctuation is used for the purpose of separating the whole number from the decimals, therefore using a point instead of a coma.
- The expression "threshold value" or "limit value", when it refers to the expression levels of the miRNA described in the present invention, refers to a reference expression level indicative of there being a probability of subject suffering asthma or a type of asthma determined as mild, moderate, or severe intermittent asthma or persistent asthma with given sensitivity and specificity if the expression levels of the patient are above said threshold levels, limit, or reference levels.
- The expression "comprising" seeks to include, but without limitation, the elements following the expression "comprising". Therefore, the use of the expression "comprising" indicates that the mentioned elements are necessary or mandatory, but that other elements are optional and may or may not be present.
- The expression "consisting of" is understood to include, and to be limited to the elements following the expression "consisting of". Therefore, the expression "consisting of" indicates that the elements mentioned are necessary or mandatory and that any other element may not be present.
- It must also be taken into account that, as it is used herein the term "kit" is not limited to any specific device and includes any device suitable for putting the invention into practice such as, but without limitation, microarrays, bioarrays, biochips, or biochip arrays.

A variety of statistical and mathematical methods for establishing the limit or threshold expression level is known in the prior art. A limit or threshold expression level for a particular biomarker can be selected, for example, based on data from the receiver operating characteristic (ROC) graphs, as described in the Examples and drawings of the present invention. One person skilled in the art will be able to see that these limit or threshold expression levels can vary, for example, shifting along the ROC graph for the particular biomarker of the present invention, to obtain different sensitivity or specificity values, thereby affecting the overall performance of the test. For example, if the objective is to have a solid diagnostic method from the clinical viewpoint, it would be necessary to try to have a high sensitivity. However, if the objective is to have a cost-effective method, it would be necessary to try to achieve a high specificity. The best limit refers to the value obtained from the ROC graph for the particular biomarker of the present invention which delivers the best sensitivity and specificity. The sensitivity and specificity values are calculated based on the threshold (limits) interval. The limit or threshold values can therefore be selected such that the sensitivity and/or specificity are at least of about 70%, and can be, for example, at least 75%, at least 80%, at least 85%, at least 90%, at least 95%, at least 96%, at least 97%, at least 98%, at least 99%, or at least 100% in at least 60% of the tested population of patients, or in at least 65%, 70%, 75%, or 80% of the tested population of patients.

Accordingly, each of the embodiments mentioned throughout the present invention is preferably performed by determining the expression levels of at least miR-185-5p in a sample isolated from a subject to be diagnosed or examined, and comparing the expression levels of said microRNA with predetermined limit or threshold values.

### DESCRIPTION

As expressed in the present invention, asthma is a chronic inflammatory disease of the respiratory tracts, whose pathogenesis involves various types of cells and inflammation mediators, is partly determined by genetic factors, and occurs with bronchial hyperresponsiveness (BHR) and varying degrees of airflow obstruction, completely or partially reversible, either spontaneously or as a result of pharmacological action. Asthma is a heterogeneous syndrome resulting from complex interactions between environmental and genetic factors. The most commonly used classifications are based on its severity and the degree of control achieved with treatment.

Table 1 describes asthma classification based on severity according to the Spanish Guideline on the Management of Asthma (GEMA 4.0) in adults:

**Table 1. Classification of asthma by severity (before treatment)**

| | Intermittent | Mild Persistent | Moderate Persistent | Severe Persistent |
|---|---|---|---|---|
| Daytime symptoms | No (2 times or less a week) | More than 2 times a week | Daytime symptoms | Continuous symptoms (several times a day) |
| Relief medication (short-acting β₂ adrenergic agonist) | No (2 times or less a week) | More than 2 times a week, but not every day | Every day | Several times a day |
| Nighttime symptoms | No more than twice a month | More than twice a month | More than once a week | Often |
| Limitations on activity | None | Some limitation | A fair degree of limitation | Considerable |
| Pulmonary function (FEV1 or PEF) Theoretical percentage | >80% | >80% | >60% - <80% | ≤60% |
| Exacerbations | None | One or none a year | Two or more a year | Two or more a year |

| | | | | |
|---|---|---|---|---|
| FEV₁: Forced expiratory volume in the first second; PEF: peak expiratory flow. | | | | |

Table 2 summarizes the classification based on asthma control according to the Spanish Guideline on the Management of Asthma (GEMA 4.0) in adults.

**Table 2. Asthma classification in adults based on asthma control**

| | WELL controlled (all of the following) | PARTIALLY controlled (any measure in any week) | POORLY controlled |
|---|---|---|---|
| Daytime symptoms | None or ≤2 times a week | >2 times a week | If ≥3 features of partially controlled asthma |
| Limitations on activity | None | Any activity | |
| Nighttime symptoms/Symptoms on waking | None | Any symptom | |
| Need for relief (rescue) medication (SABA) | None or ≤2 times a week | >2 times a week | |
| Pulmonary function | >80% theoretical value | <80% theoretical value | |
| - FEV₁, | | | |
| - PEF | >80% best personal value | <80% best personal value | |
| Exacerbations | None | ≥1/year | ≥1 in any week |

| | | | |
|---|---|---|---|
| FEV₁: Forced expiratory volume in the first second; PEF: peak expiratory flow; ACT: asthma control test. SABA: short-acting β₂ adrenergic agonist. | | | |

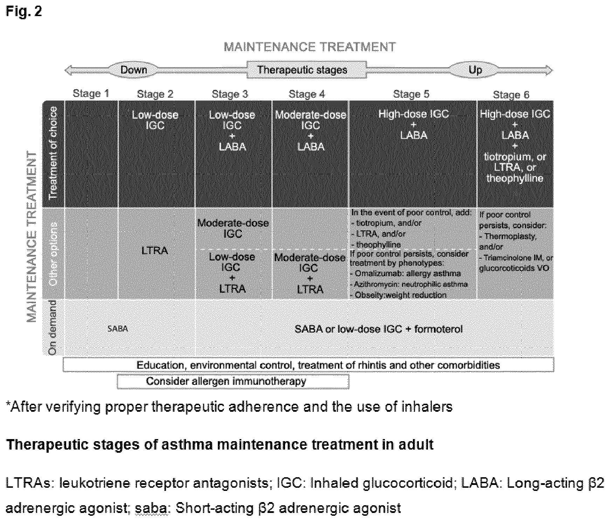

Current treatments for asthma and the stratification thereof for the different treatments according to the Spanish Guideline on the Management of Asthma (GEMA 4.0) in adults are shown in Figure 2, where it can be verified that said treatments are selected from the list comprising the following (it must be noted that this list is not exhaustive): leukotriene receptor antagonists, inhaled glucocorticoids, long-acting beta 2 adrenergic agonists, and short-acting beta 2 adrenergic agonists, as well as treatments with anti-IL 5 or anti-IL4/IL-13.

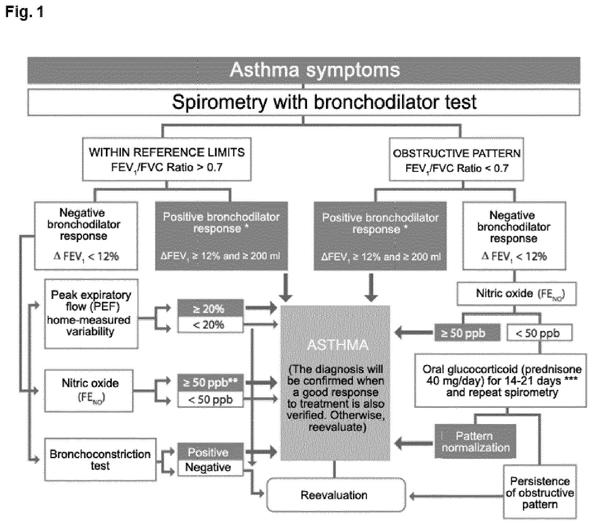

The diagnosis of asthma is currently based on clinical manifestations and on demonstrating the reversible obstruction of respiratory tracts following an algorithm similar to the proposed in Figure 1.

With respect to the asthma biomarkers known to date, the following classification is considered:
1. Very few tools are included among non-aggressive or non-invasive biomarkers. Determination of **eosinophils** in blood is one of said tools. In this sense, eosinophils in blood (> of 300 eosinophils/µL) is a finding which, together with the symptoms and variability of bronchial obstruction, backs the diagnosis of asthma. However, not all asthma occurs with eosinophilia. The absence of eosinophilia is also a marker indicating a good response to treatment with both inhaled and systemic steroids, and with biological therapies such as treatment with anti-IL 5 or anti-IL4/IL-13. It is also considered as a Th2-type immune activation marker.
2. Determination of the inflammatory component by means of studying cellularity by flow cytometry in the induced sputum from patients is also used as a method which contributes to the diagnosis and follow-up of the patient with asthma.
3. Determination of **FeNO** is used to back the diagnosis of asthma, as well as a good response to the same medications determining eosinophilia. It is a marker indicating Th2 immune pathway activation. Its specificity increases when the cut-off point is at more than 50 ppb, but, in contrast, its sensitivity at this cut-off point is lower.
4. Research is being conducted for **periostin** in blood as an asthma biomarker. It depends on Th2 immune activation and other mechanisms. Its overall sensitivity is relatively low because, like with the previous markers, it only detects Th2 immune pathway activation and there are many patients with asthma whose bronchial inflammation depends on other pathways.
5. Other biomarkers currently under development include determination of dipeptidyl peptidase-4 in blood, determination of exhaled air temperature, or determination of pH in exhaled air condensate, as well as determination of volatile organic compounds (VOCs) in exhaled air; said biomarkers may also be useful in the diagnosis of asthma.
6. With respect to markers for indicating severity in asthma, the most widely used include determination of eosinophils in blood or in sputum. Some pulmonary function tests, such as spirometry itself or determination of residual volume, can help to determine asthma severity. Research is being conducted for new imaging techniques seeking to separate asthma into its phenotypes by its potential severity.

On the clinical level and in relation to diagnostic difficulty, the differential presence of certain miRNAs would have the advantage of being a marker with higher sensitivity, backing the diagnosis of asthma in a manner independent of the presence of inflammation exclusively mediated by Th2, unlike the actions of other markers (eosinophils, exhaled nitric oxide, periostin), given that not all the patients with asthma (about 50%) present an overexpression of this inflammation-causing pathway.

The present study shows that, among other miRNAs, miR-185-5p has higher relative expression levels (2^{-ΔCt}; where ΔCt = Ct of the miRNA of interest (miR-185-5p) - Ct of the endogenous miRNA) in patients with asthma compared with healthy individuals (Figure 4).

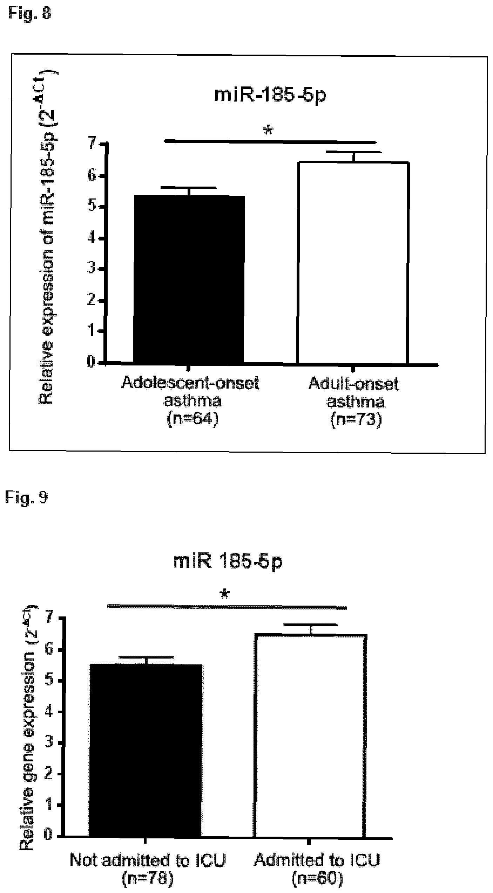

Furthermore, not only are the expression levels of miR-185-5p different between healthy individuals and individuals with asthma, but also between healthy individuals and each of the subgroups of individuals with (intermittent, mild persistent, moderate persistent, and severe persistent) asthma. The results furthermore show that the last two stages of severity, i.e., the moderate and severe persistent stages, show a progressive increase or escalation of the expression thereof compared with mild intermittent and persistent stages (Figure 5 and Figure 9). Figure 9 strengthens this conclusion as it shows how those patients with asthma who have been admitted at least once to intensive care unit or ICU have higher expression levels of miR-185-5p than individuals with asthma. This strengthens the conclusion that not only are the expression levels of miR-185-5p different between healthy individuals and individuals with asthma, but said levels are also a clear indicator of asthma severity.

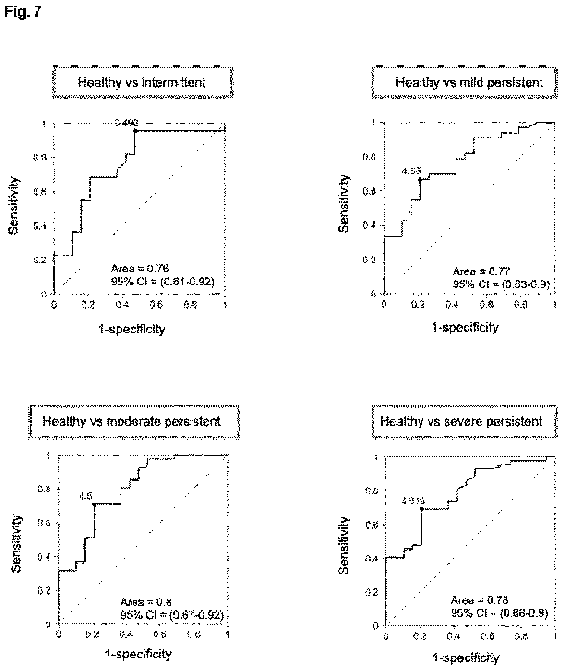

To characterize the possibility of using the expression of miR-185-5p in biological blood, serum, or plasma samples as an asthma biomarker, ROC curves comparing the expression levels of miR-185-5p between healthy individuals and individuals with asthma were plotted. As can be verified in the results provided in the present specification, miR-185-5p was capable of distinguishing healthy individuals from patients with asthma (Figure 6), and healthy individuals from each of the subgroups of individuals with asthma (Figure 7). The results of the relative expression levels of each miRNA were analyzed by means of a ROC curve to determine if this difference in expression between healthy individuals and individuals with asthma had a cut-off point that was capable of distinguishing them from one another and of classifying RNAs. The defined cut-off point is the point at which the Youden's index was the highest (defined as sensitivity plus specificity minus 1), so other cut-off points could be defined according to the sensitivity or specificity requirements needed according to the application.

Therefore, the expression levels of miR-185-5p in biological blood, serum, or plasma biological samples collected in a non-invasive manner may provide a biomarker useful for diagnosing or selecting patients with asthma and patients with no asthma, backing the diagnosis of asthma in a manner independent of the presence of inflammation exclusively mediated by Th2, and determining the potential asthma severity in those patients with asthma.

Based on the foregoing data, it is understood that this biomarker will be very useful in the diagnosis of patients with asthma regardless of the asthma endotype that is present. miR-185-5p is furthermore a possible regulator of genes involved in allergy response such as periostin, since the *POSTN* gene is a theoretical target gene (not verified by means of experimental methods) according to bioinformatic tools for predicting the targets of miRNA RNA according to the sequence thereof (miRSystem).

In summary, the present specification identifies, for the first time, miR-185-5p, commonly deregulated in biological samples collected in a minimally invasive manner, such as a plasma sample, blood sample, cerebrospinal fluid sample (CSF), or a serum sample, as a biomarker useful in the diagnosis/prognosis/selection of patients suffering asthma.

A first aspect of the invention therefore relates to an *in vitro* method for selecting subjects with the risk of suffering asthma, particularly distinguishing mild intermittent or persistent asthma from moderate or severe persistent asthma, which comprises: (a) measuring the expression pattern or level of at least miR-185-5p obtained from a biological sample isolated from the subjects to be selected; and (b) comparing said expression pattern or level of at least miR-185-5p of the subjects to be selected with a pre-established expression pattern or level, said pattern are preferably the expression levels of miR-185-5p in healthy individuals, where the overexpression of at least miR-185-5p is indicative of said subject having a risk of suffering asthma, particularly distinguishing the risk of suffering mild intermittent or persistent asthma from suffering moderate or severe persistent asthma. In this sense, it is important to understand that higher relative expression levels of miR-185-5p (2^{-ΔCt}; where ΔCt = Ct of the miRNA of interest (miR-185-5p) - Ct of the endogenous miRNA) in patients with asthma compared with healthy individuals (Figure 4) are indicative of said subject having asthma or having a risk of suffering asthma. Furthermore, not only are the expression levels of miR-185-5p different between healthy individuals and individuals with asthma, but also between healthy individuals and each of the subgroups of individuals with (intermittent, mild persistent, moderate, and severe) asthma, where the expression of miR-185 in individuals with moderate and severe persistent asthmas shows a progressive increase or escalation compared with individuals with mild intermittent and persistent asthmas. It is therefore important to understand that not only are the higher relative expression levels of miR-185 (2^{-ΔCt}; where ΔCt = Ct of the miRNA of interest (miR-185-5p) - Ct of the endogenous miRNA) in patients with asthma compared with healthy individuals indicative of said subject having asthma or having a risk of suffering asthma, but also indicative of the level of asthma severity that said patient suffers or may suffer.

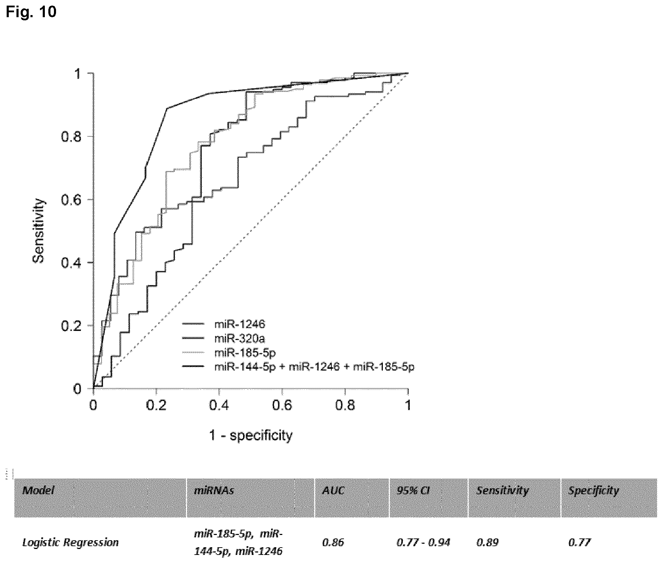

Furthermore, as shown in Figures 10 and 11 it is demonstrated in a logistic regression model that the joint expression of the miRNAs, miR-185-5p, miR-144-5p, and miR-1246, or the joint expression of miRNAs, 185-5p, miR-320a, and miR-320b, increase the sensitivity and specificity of asthma diagnosis compared with each of these miRNAs taken individually.

Therefore, a preferred embodiment of the first aspect of the invention relates to an *in vitro* method for selecting subjects with the risk of suffering asthma, particularly distinguishing mild intermittent or persistent asthma from moderate or severe persistent asthma, which comprises: (a) measuring the expression pattern or level of at least miR-185-5p, miR-144-5p, and miR-1246 or of at least the miRNAs, 185-5p, miR-320a, and miR-320b, obtained from a biological sample isolated from the subjects to be selected; and (b) comparing said expression pattern or level of at least miR-185-5p, miR-144-5p, and miR-1246, or miR-185-5p, miR-320a, and miR-320b of the subjects to be selected with a pre-established expression pattern or level, said pattern are preferably the expression levels of miR-185-5p, miR-144-5p, and miR-1246, or miR-185-5p, miR-320a, and miR-320b in healthy individuals, where the overexpression of at least miR-185-5p, miR-144-5p, and miR-1246, or miR-185-5p, miR-320a, and miR-320b is indicative of said subject having a risk of suffering asthma, particularly distinguishing the risk of suffering mild intermittent or persistent asthma from suffering moderate or severe persistent asthma. In this sense, it is important to understand that higher relative expression levels of miR-185, miR-144-5p, and miR-1246, or of miR-185-5p, miR-320a, and miR-320b in patients with asthma compared with healthy individuals (Figure 10) are indicative of said subject having asthma or having a risk of suffering asthma. Furthermore, not only are the expression levels of miR-185-5p, miR-144-5p, and miR-1246, or miR-185-5p, miR-320a, and miR-320b different between healthy individuals and individuals with asthma, but also between healthy individuals and each of the subgroups of individuals with (intermittent, mild persistent, moderate, and severe) asthma, where the individuals with moderate and severe asthmas show a progressive increase or escalation of each of these miRNAs compared with individuals with mild intermittent and persistent asthmas. It is therefore important to understand that not only are the higher relative expression levels of miR-185, miR-144-5p, and miR-1246, or miR-185-5p, miR-320a, and miR-320b in patients with asthma compared with healthy individuals indicative of said subject having asthma or having a risk of suffering asthma, but also indicative of the level of asthma severity that said patient suffers or may suffer.

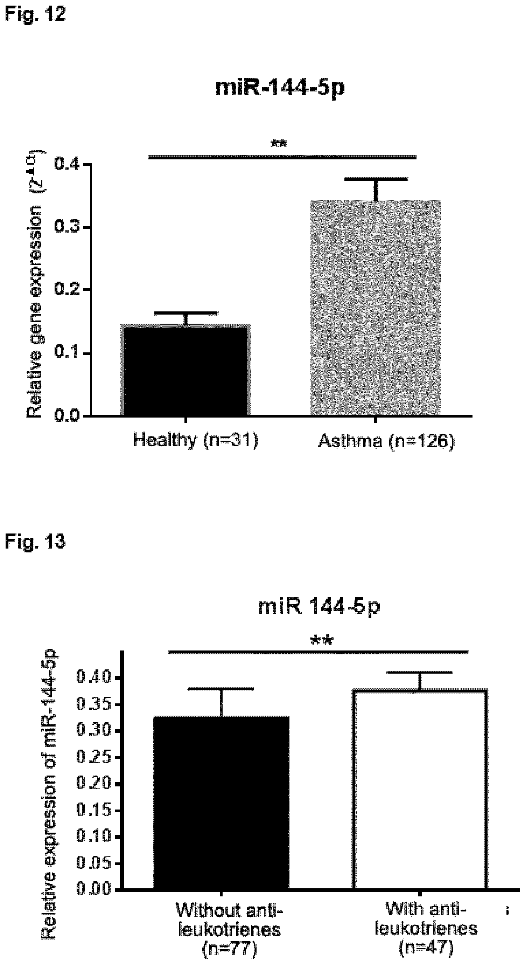

On the other hand, and in a first alternative aspect with respect to the foregoing, as illustrated in Figure 12, miR-144-5p is overexpressed in patients with asthma compared with healthy individuals. Therefore, this first aspect of the invention relates to an *in vitro* method for selecting subjects with the risk of suffering asthma, which method comprises: (a) measuring the expression pattern or level of at least miR-144-5p obtained from a biological sample isolated from the subjects to be selected; and (b) comparing said expression pattern or level of at least miR-144-5p of the subjects to be selected with a pre-established expression pattern or level, said pattern are preferably the expression levels of miR-144-5p in healthy individuals, where the overexpression of at least miR-144-5p is indicative of said subject having a risk of suffering asthma. In this sense, it is important to understand that higher relative expression levels of miR-144-5p in patients with asthma compared with healthy individuals (Figure 12) are indicative of said subject having asthma or having a risk of suffering asthma.

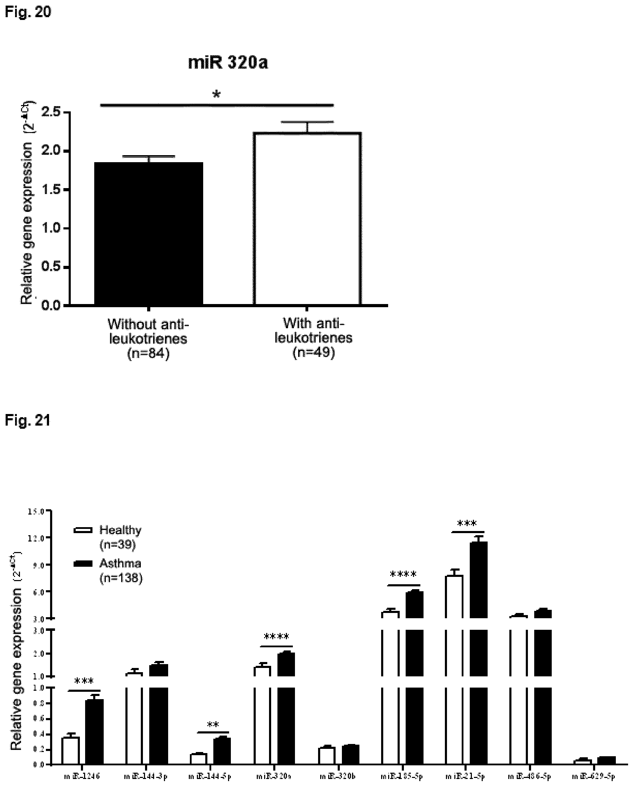

Furthermore, the expression levels of miR-144-5p are not only different between healthy individuals and individuals with asthma. The individuals with more severe asthma according to the administered treatments show an escalation or increase of this miRNA. The latter is clearly illustrated in Figures 13, 14, and 20 showing how patients treated with anti-leukotrienes have higher relative expression levels of miR-144-5p than non-treated patients, and how miR-144-5p is overexpressed in patients with asthma who have been subjected to treatment with bronchodilators.

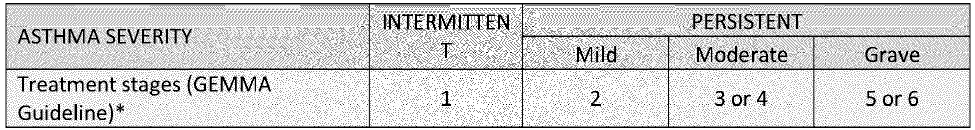

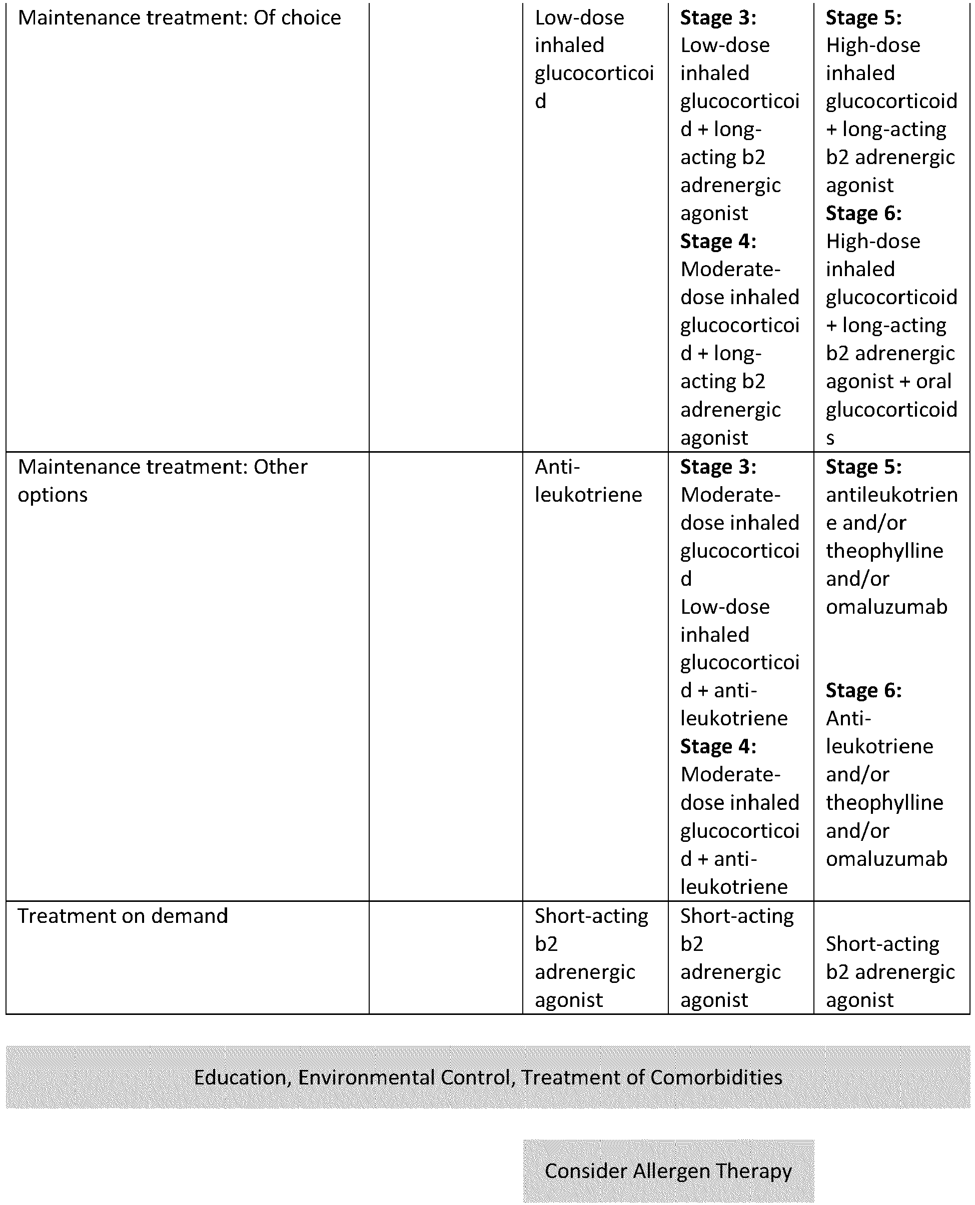

In this sense and as inferred from the table above, treatments with bronchodilators and/or with anti-leukotrienes identified in Figures 13, 14, and 20 chronically denote a persistent asthma. These drawings therefore back the fact that not only are the higher relative expression levels of miR-144-5p in patients with asthma compared with healthy individuals indicative of said subject having asthma or having a risk of suffering asthma, but also are indicative of the level of asthma severity that said patient suffers or may suffer.

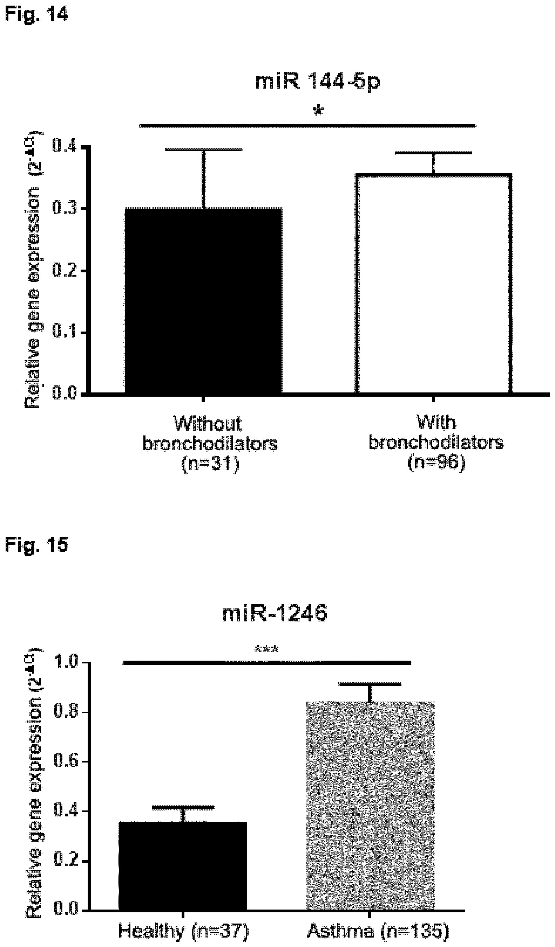

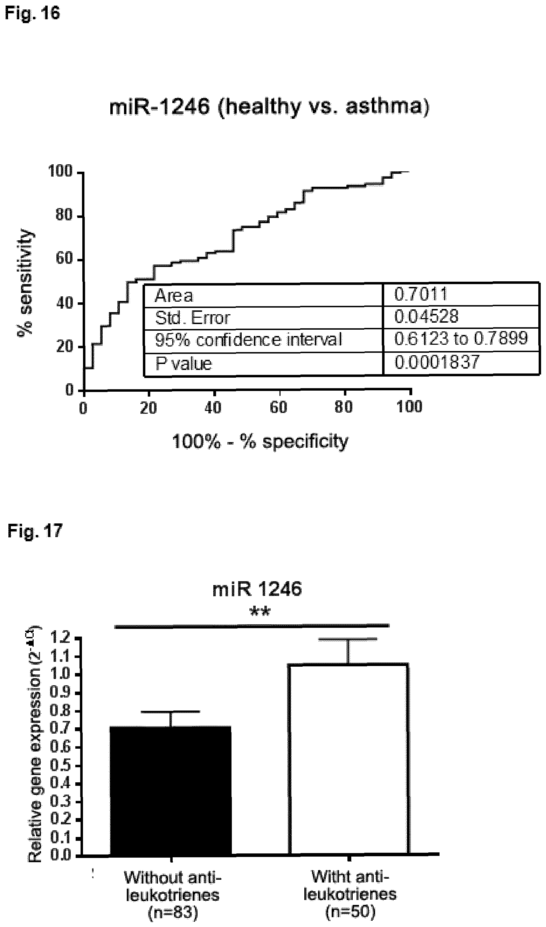

Furthermore, in another first alternative aspect with respect to the foregoing, as illustrated in Figures 15 and 16, the miR-1246 is overexpressed, with an AUC greater than 0.7, in patients with asthma compared with healthy individuals. Therefore, this first aspect of the invention relates to an *in vitro* method for selecting subjects with the risk of suffering asthma, which method comprises: (a) measuring the expression pattern or level of at least miR-1246 obtained from a biological sample isolated from the subjects to be selected; and (b) comparing said expression pattern or level of at least miR-1246 of the subjects to be selected with a pre-established expression pattern or level, said pattern are preferably the expression levels of miR-1246 in healthy individuals, where the overexpression of at least miR-1246 is indicative of said subject having a risk of suffering asthma. In this sense, it is important to understand that higher relative expression levels of miR-1246 in patients with asthma compared with healthy individuals (Figure 15) are indicative of said subject having asthma or having a risk of suffering asthma.

Furthermore, the expression levels of miR-1246 are not only different between healthy individuals and individuals with asthma. Individuals with more severe asthma according to the administered treatments show an escalation or increase of this miRNA. The latter is clearly illustrated in Figure 17. Patients treated with anti-leukotrienes have higher relative expression levels of miR-1246 than non-treated patients.

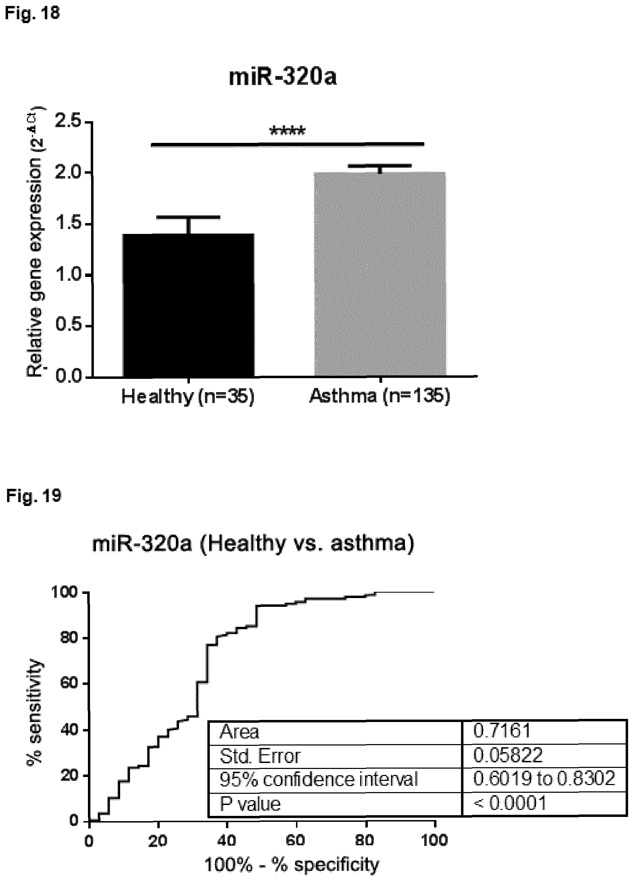

Furthermore, and in still another first alternative aspect with respect to the foregoing, as illustrated in Figures 18 and 19, miR-320a is overexpressed in patients with asthma compared with healthy individuals. Therefore, this first aspect of the invention relates to an *in vitro* method for selecting subjects with the risk of suffering asthma, which method comprises: (a) measuring the expression pattern or level of at least miR-320a obtained from a biological sample isolated from the subjects to be selected; and (b) comparing said expression pattern or level of at least miR-320a of the subjects to be selected with a pre-established expression pattern or level, said pattern are preferably the expression levels of the miR-320a in healthy individuals, where the overexpression of at least miR-320a is indicative of said subject having a risk of suffering asthma. In this sense, it is important to understand that higher relative expression levels of miR-320a in patients with asthma compared with healthy individuals (Figure 18 and 19) are indicative of said subject having asthma or having a risk of suffering asthma.

A second aspect of the invention relates to the *in vitro* method for the diagnosis/prognosis of a subject who is suspected of suffering asthma, which method comprises steps a) and b) of any of the methods of the first aspect of the invention or of any of the preferred embodiments thereof, and optionally (c) confirming the presence of asthma and/or the severity of the disease and/or selecting individuals developing asthma during adulthood or during adolescence by means of a clinical examination, particularly by using any of the additional biomarkers (see the aforementioned miRNAs).

A third aspect of the invention relates to a method for obtaining useful data for the *in vitro* diagnosis/prognosis of asthma, distinguishing subjects with less severe asthma from those with more severe asthma, particularly distinguishing mild intermittent or persistent asthma from moderate or severe persistent asthma, which method comprises steps a) and b) of any of the methods of the first aspect of the invention or of any of the preferred embodiments thereof.

On the other hand, as described above, the results set forth in the Examples of the present invention show that, at least in the last two stages of severity, i.e., in moderate and severe persistent stages, the expression of miR-185 show a progressive increase or escalation compared with patients with mild intermittent and persistent asthmas. This in turn will allow using the relative expression levels of miR-185 (2^{-ΔCt}; where ΔCt = Ct of the miRNA of interest (miR-185-5p) - Ct of the endogenous miRNA) in patients with asthma to be diagnosed compared with the known expression levels of this same miRNA in patients with asthma diagnosed with different levels of asthma severity, establishing the level of severity of the patient with asthma, and therefore serving as a tool which helps diagnosing the degree of severity of the disease in a patient with asthma. Furthermore, Figure 11 shows how in a Random Forest model, the joint expression levels of the miRNAs, miR-320a, miR-185-5p, and miR-144-5p are capable of improving the capacity of the expression levels of miR.185, taken individually, to establish the level or stage of severity of a patient with asthma, significantly improving the classification of said patient who has the risk of suffering asthma or is suffering asthma in any of the following categories: healthy individual, individual with intermittent asthma, mild persistent asthma, moderate persistent asthma, or severe persistent asthma.

Therefore, a fourth aspect the invention refers to an *in vitro* method for classifying a subject with the risk of suffering asthma into one of the following categories: healthy individual, individual with intermittent asthma, mild persistent asthma, moderate persistent asthma, or severe persistent asthma, which method comprises: (a) measuring the expression pattern or level of at least miR-185-5p, preferably in combination with the miRNAs, miR-320a and miR-144-5p, obtained from a biological sample isolated from the subject to be classified; and (b) comparing said expression pattern or level of at least miR-185-5p, preferably in combination with the miRNAs, miR-320a and miR-144-5p, of the subject to be classified with a pre-established expression pattern or level for each of said markers, where the difference in the expression of at least miR-185-5p, preferably in combination with miR-320a and miR-144-5p, with respect to said pre-established expression pattern or level allows classifying said subject with the risk of suffering asthma as healthy individual, individual with intermittent asthma, mild persistent asthma, moderate persistent asthma, or severe persistent asthma.

A fifth aspect of the invention relates to an *in vitro* method for monitoring response to a therapy or for monitoring the progression of asthma, distinguishing subjects with less severe asthma from those with more severe asthma, particularly distinguishing mild intermittent or persistent asthma from moderate or severe persistent asthma, in a subject suffering said disease, which method comprises steps a) and b) of any of the methods of the first aspect of the invention or of any of the preferred embodiments thereof.

A sixth aspect of the invention relates to a method for treating subjects suffering asthma, distinguishing subjects with less severe asthma from those with more severe asthma, particularly distinguishing mild intermittent or persistent asthma from moderate or severe persistent asthma, which method comprises steps a) and b) of any of the methods of the first aspect of the invention or of any of the preferred embodiments thereof, and (c) treating the patient who has been diagnosed with asthma, particularly distinguishing mild intermittent or persistent asthma from moderate or severe persistent asthma. The patient is preferably treated with leukotriene receptor antagonists, inhaled glucocorticoids, long-acting beta 2 adrenergic agonists, or short-acting beta 2 adrenergic agonists, depending on the diagnostic result. Needless to say that this treatment will be determined according to asthma severity and according to the regimens normally established for said stage of severity, particularly the treatment regimens including the doses and the number and route of administration, as well as the types of treatments are established as described in Figure 2.

In another preferred embodiment of the method according to any of the methods of the first to the fifth aspects of the invention or of any of the preferred embodiments thereof, the biological sample is selected from the group consisting of a biological sample collected in a minimally invasive manner from the subjects to be selected, such as a plasma sample, a blood sample, a cerebrospinal fluid sample (CSF), or a serum sample.

In another additional preferred embodiment of the method according to any of the methods of the first to the sixth aspects of the invention or of any of the preferred embodiments thereof, the subject is a human subject.

A seventh aspect of the invention relates to the use of a kit comprising reagents which detect biomarkers for determining a differential expression level of any of the biomarkers or any of the combinations of biomarkers mentioned in the methods of the first aspect of the invention or in any of the preferred embodiments thereof, where the overexpression or differences in the expression of any of these biomarkers is indicative of a risk of developing asthma, distinguishing subjects with less severe asthma from those with more severe asthma, particularly distinguishing mild intermittent or persistent asthma from moderate or severe persistent asthma, for diagnosing the risk of developing asthma *in vitro*, distinguishing subjects with less severe asthma from those with more severe asthma, particularly distinguishing mild intermittent or persistent asthma from moderate or severe persistent asthma.

A preferred embodiment of the seventh aspect of the invention relates to the use of the kit, where said kit comprises reagents for determining a differential expression level of at least miR-185-5p for diagnosing the risk of having asthma *in vitro*, particularly distinguishing mild intermittent or persistent asthma from moderate or severe persistent asthma.

In a preferred embodiment of the use of the kit according to the seventh aspect of the invention or of any of the preferred embodiments thereof, said reagents are selected from the group consisting of all or at least one of the following: i) primers specific for the miRNA or miRNA combinations as defined in any of said aspects or preferred embodiments which are capable of producing primer-linked miRNA sequences; ii) reverse transcription means for producing complementary DNA (cDNA) from the primer-linked miRNA sequences of i); iii) means such as primers capable of amplifying the cDNAs derived from the primer-linked miRNA sequences as defined in i); iv) means for transcribing the amplified cDNAs to produce sense target RNA; and v) a population of antisense miRNA probes capable of detecting the sense target RNA of iv).

On the other hand, it must be mentioned that the present invention is preferably carried out in plasma or serum samples obtained from the patients. On the other hand, it is important to highlight that obtaining serum or plasma preparations from blood comprises several steps performed by technicians: in the case of serum, blood is left to clot by leaving it to settle at room temperature, the clot is removed by centrifugation, and the supernatant which is called serum is isolated. In the case of plasma, centrifugation is also required. Furthermore, after the centrifugation process, it is important to immediately transfer the liquid component (serum or plasma) to a clean tube. The samples are kept at 2-8°C during handling. If the serum or plasma is not analyzed immediately, they must be split into aliquots, stored, and transported at -80°C or at a lower temperature. It is important to prevent freezing-thawing cycles because they are damaging to many serum components. Hemolyzed, icteric, or lipemic samples may invalidate certain tests.

The inventions described in an illustrative manner herein can be suitably put into practice in the absence of any element or elements, limitation or limitations, not specifically disclosed herein. In this sense, for example, the expressions "comprising", "including", "containing," etc. must be considered in a broad sense and without any limitation. Furthermore, the terms and expressions used herein have been used with descriptive and non-limiting purposes, and the use of said terms and expressions does not at all seek to exclude any equivalent of the shown and described features or parts thereof, but rather recognizes that various modifications within the scope of the claimed invention are possible. It must therefore be understood that, although the present invention has been specifically disclosed by means of preferred embodiments and optional features, those skilled in the art can resort to modifications and variations of the invention incorporated and disclosed herein, and said modifications and variations are considered within the scope of this invention.

The invention has been described herein in a broad and general sense. Each of the species and more general subgenus groupings included in the general description also forms part of the invention. This includes the general description of the invention with a negative or limiting condition which eliminates any subject-matter of the genus, regardless of whether the eliminated material has been specifically mentioned herein.

Other embodiments are included in the following claims and non-limiting examples. Furthermore, when the features or aspects of the invention are described in reference to groups, those skilled in the art will recognize that the invention is also described in reference to any individual member or to any subgroup of members of the group.

### Examples

### MATERIALS AND METHODS

### - Patients and sample collection

The samples that were used in the present study came from a biobank sample collection of individuals with asthma created in 2006 within the framework of Breath Network (Red Respira) funded by Carlos III Health Institute (*Instituto de Salud Carlos III* - ISCIII). Individuals with asthma were selected from patients who visited the Allergology and Pneumology Department of the hospitals forming part of the Network (Madrid, Canary Islands, and Barcelona). Healthy individuals who participated in the study were the staff from the Allergy and Immunology Department of the research area of Fundación Jiménez Díaz Health Research Institute (*Instituto de Investigación Sanitaria de la Fundación Jiménez Díaz* - IIS-FJD).

Biological peripheral blood samples were obtained by venipuncture always after all the individuals confirmed their participation in the study and signed the informed consent. Said consent provides explanation to the patient concerning the project to be undertaken and its prior approval by the Ethics Committee of the IIS-FJD according to the principles of the Declaration of Helsinki. Likewise, the patients included in said study were asked about the age of asthma onset and the ages were collected (value from age 1 to age 99), being grouped into two groups according to the age of asthma onset: adolescent-onset asthma (from age 1 to age 17) and adult-onset asthma (from age 18 to age 99).

The selection of the subjects of study was restricted to a series of inclusion criteria that are described in detail below for each group of individuals.
- Healthy individuals. Individuals who are non-smokers, without any history of asthma or allergy diseases, and/or BENA.
- Individuals with asthma. All patients from this group were diagnosed as individuals with asthma following the criteria established according to the American Thoracic Society. These individuals showed an improvement in forced respiratory volume greater than 12% for the first 10 minutes after the administration of inhaled terbutaline, having a positive methacholine test (PC20 less than 16 mg/ml (particularly less than <8 mg/ml), which implies airway hyperresponsiveness. Patients with asthma were classified according to the severity of their asthma phenotype into 4 subgroups: individuals with intermittent asthma, individuals with mild persistent asthma, individuals with moderate persistent asthma, and individuals with severe persistent asthma, following the standards created by the GEMA Guideline, a guideline elaborated at the state level for asthma management (GEMA 4.0. Spanish Guideline on the Management of Asthma). Both atopic and non-atopic patients were included.

### miRNA extraction

For extracting miRNAs from biological fluid samples, the miRCURY RNA Isolation Kit-Biofluids (Bionova, Exiqon, Copenhagen, Denmark) was used, and extraction was performed following manufacturer's instructions. Briefly, the serum samples are thawed and centrifuged at 3000 g for 5 minutes. Next, 200 µL of supernatant are taken and transferred to a new tube, where said 200 µL of supernatant will be the initial sample volume to be worked on. Next, there are added 60 µL of lysis solution BF to which there has been previously added 1 µL of Spike-in mix (miRCURY LNA™ Universal RT microRNA PCR, RNA Spike-in kit) containing three synthetic miRNAs (UniSp2, UniSp4, UniSp5) which are at different concentrations and serve as a control to ensure that extraction has been performed correctly. It is incubated for 1 to 3 minutes at room temperature (RT). 20 µL of protein precipitation solution BF are added. It is stirred with vortex and incubated for 1 minute at RT. It is centrifuged for 3 minutes at 11000 g and the supernatant is taken to which 270 µL of isopropanol are added for nucleic acid separation. The sample and isopropanol volume is passed through a column of the kit by means of centrifugation for 30 seconds at 11000 g, after a 2-minute sample incubation in the column. Nucleic acids are trapped in the column, so they must then be washed by means of using different volumes of wash solution BF I and II. After washing, the membrane of the column to which the nucleic acids bound is treated with DNAse for 15 minutes and the preceding washings are repeated. Finally, the column is removed and placed in a 1.5 ml Eppendorf. The membrane is incubated with 50 µL of H₂O, RNAse-free RNA, for 1 minute, and centrifuged at 11000 g for 1 minute. The (miRNA-enriched) RNA elution volume is 50 µL of RNA that would be stored at -80°C until use.

### - miRNA expression analysis by means of real time qRT-PCR

cDNA reverse transcription is performed by means of the Universal cDNA Synthesis kit II (Bionova, Exiqon, Copenhagen, Denmark) following manufacturer's instructions. Briefly, a mixture of enzymes and probes, which is then added to each PCR tube, is prepared. This mixture consists of 2 µL of reaction buffer, 4.5 µL of RNAse-free H₂O, and 1 µL of reverse reverse transcriptase enzyme for each sample. Finally, 2 µL of sample (RNA) will be added. There is furthermore a need to add a control for ensuring proper performance of reverse transcription; which control will be the synthetic RNA spike-in (Sp6) from which 0.5 µL are added per tube of sample.

Finally, qRT-PCR is performed in a Veriti Thermal Cycler (Applied Biosystem, Warrington, United Kingdom). The temperature and time program to be applied is as follows:
∘ 60 minutes at 42°C
∘ 5 minutes kept at 95°C
∘ Keep at 4°C or freeze at -20°C

To perform real time qPCR or PCR, the cDNA obtained from the reverse transcription of the miRNAs extracted from serum samples is used. The cDNA must be diluted 40 times (a 1:40 dilution) for use in qPCR. A mixture which must be made up of enzymes, fluorescent nucleotides, and template cDNA is prepared. To that end, the ExiLENT SYBR® Green master mix kit (Bionova) and probes specific for each miRNA to be studied, which will be microRNA LNA™ PCR primer set probes (Bionova), are used.

Each of these mixtures is prepared in a well of a 96-well plate, i.e., 96-well PCR microplate for LC480 (Roche, Basel, Switzerland), and consists of 5 µL of PCR Master mix, 1 µL of the specific primer of the miRNA, and 4 µL of the cDNA diluted at a 1:40 dilution. PCR is carried out in a Light Cycler 96 (Roche). The protocol to be applied is as follows :
∘ Pre-incubation for 10 minutes at 95°C
∘ Two-step amplification, the first one at 95°C for 10 seconds and then at 60°C for 1 minute, performing a total of 45 cycles
∘ Three-step fusion, the first one at 95°C for 5 seconds, 65°C for 1 minute, and 97°C for 1 second
∘ Cooling at 40°C for 10 seconds.
∘ Keep at -20°C until use.

The results are analyzed using the LightCycler® 96 SW 1.1 computer software (Roche). The miRNAs recommended by the manufacturer are used as endogenous controls or housekeeping miRNAs: hsa-miR-103a-3p and hsa-miR-191-5p. The gene expression of each miRNA is analyzed for each sample using the Ct (*Cycle Threshold*) value obtained from qPCR. The endogenous control expression results obtained by means of qPCR are shown as the cycle thresholds (Ct) required to attain specific fluorescence, given by the amount of cDNA in that cycle. In this sense, since qPCR amplifies the cDNA in an exponential manner, the higher the initial concentration of cDNA synthesized from a specific miRNA, the less cycle will be needed to attain the fluorescence threshold. Relative gene expression is calculated in the following manner: 2^{-ΔCt}; where ΔCt = Ct of the miRNA of interest (miR-185-5p) - Ct of the endogenous miRNA.

### Data analysis

Comparisons between groups of patients are carried out by means of the Student's T parametric test when the patients show values complying with a normal or Gaussian distribution, whereas the Mann-Whitney test is performed for samples that fail the normality test (non-parametric samples). The ANOVA test is performed with a subsequent Bonferroni statistical test for comparisons between more than two groups, if they comply with a normal distribution, or non-parametric test for samples that do not show a normal distribution. For non-parametric samples, the Kruskal-Wallis test is performed with Dunn's post-hoc test. Values are determined as statistically significant starting from a p-value of less than 0.05 (p<0.05). The results of the comparisons of expression among miRNAs are shown with the mean value (±) the standard error of the mean (SEM) and clinical data with the mean and standard deviation (SD). Statistical calculations and graph plotting are carried out using the GraphPad Instat 3 and GraphPad Prism 6 programs (GraphPad Software Inc., San Diego, United States). ROC (Receiver Operating Characteristic) curves for defining biomarkers are plotted, taking as positive values those the area under the curve (AUC) of which is greater than 0.7. These curves are used in health to define disease markers, taking the curves having high sensitivity and specificity values the area under the curve of which is >0.7. The ROC curves show on the Y-axis sensitivity values of 0 to 1, the value of 1 being that of the highest sensitivity, and on X-axis values of 1-specificity, so the higher the specificity, the smaller the X value will be. A perfect curve would be defined by sensitivity and specificity values of 1 (so the values on the X-axis being better the closer they are to 0). A cut-off point which is the point where it will have the best sensitivity and specificity characteristics required for use as a biomarker will be defined based on the curve. This cut-off point is the value of the curve that is the closest to the top left corner of the graph, i.e. the point representing a larger area under the curve.

### Example 1. The expression profile of the analyzed miRNA distinguishes healthy subjects from individuals suffering asthma

The present study shows that miR-185-5p has higher relative expression levels (2^{-ΔCt}; where ΔCt = Ct of the miRNA of interest (miR-185-5p) - Ct of the endogenous miRNA) in patients with asthma compared with healthy individuals (Figure 4). Furthermore, not only are the expression levels of miR-185-5p different between healthy individuals and individuals with asthma, but also between healthy individuals and each of the subgroups of individuals with (intermittent, mild persistent, moderate, and severe) asthma. The results furthermore show that the last two stages of severity, i.e., the moderate and severe persistent stages, show a progressive increase or escalation of the expression thereof compared with mild intermittent and persistent stages (Figure 5).

To characterize the possibility of using the expression of miR-185-5p in serum as an asthma biomarker, ROC curves comparing the expression levels of miR-185-5p between healthy individuals and individuals with asthma were plotted. MiR-185-5p was capable of distinguishing healthy individuals from patients with asthma (Figure 6), and healthy individuals from each of the subgroups of individuals with asthma (Figure 7). The results of the ROC curves show that the expression levels of miR-185-5p are not only higher in individuals with asthma with respect to healthy individuals, but rather starting from a specific cut-off point defined by a gene expression level, they can distinguish healthy individuals from individuals with asthma. The defined cut-off point is the point at which the Youden's index is the highest (defined as sensitivity plus specificity minus 1), so other cut-off points could be easily defined.

Another result to be taken into account is the greater expression of this miRNA in patients having adult-onset asthma (>age 18) compared with those having adolescent-onset asthma. Adult-onset asthma is usually more serious than adolescent-onset asthma which may subside or even disappear over time, whereas adult-onset asthma stays on (Figure 8). This result provides a correlation between the expression levels of this miRNA and more severe asthma.

In any case, one of the main features of this biomarker is the capacity of distinguish healthy individuals from patients with asthma according to its relative expression levels with respect to a threshold level, under specific sensitivity and specificity. Unlike other asthma-associated biomarkers such as eosinophilia, periostin levels, etc., which only distinguish healthy individuals from individuals with asthma when they have Th2-associated asthma, the miR-185-5p biomarker is expressed in individuals with asthma regardless of whether or not their asthma is associated with Th2. For that reason, miR-185-5p is in an excellent asthma-associated biomarker that does not depend on the patient with asthma having Th2-type inflammation for its detection, but rather is a marker having a stronger link to asthma.

Furthermore, given that the expression thereof increases according to patient severity, it is very possible that this biomarker is associated with asthma inflammation and can therefore be an important marker for determining asthma severity in the patient, and that it serves as of a diagnostic tool for determining when an individual will develop an asthmatic process, distinguishing if said development will take place throughout or before adulthood.

It could therefore be a marker for determining potential asthma severity, also in a manner independent of the Th2 pathway.

### Example 2

This example illustrates the materials and methods as well as the results of the experiments which brought about the results illustrated in Figures 10 and 11.

A logistic regression model was carried out for determining the possibility of using a combination of several miRNAs for distinguishing asthma conditions from healthy conditions.

Two Random Forest models were developed, where 90% of the samples were used to create a classification algorithm, and the remaining 10% was used as a test sample to determine the classification power of the algorithm previously created. Both Random Forest models were carried out using the relative expression (2-ΔCt) of the miRNAs: miR-1246, miR-144-3p, miR-144-5p, miR-320a, miR-320b, Mir-185-5p, miR-486-5p, miR-629-5p, and miR-21-5p, a cross-validation being performed ten times. From the miRNAs used for testing the model, three miRNAs having greater weight in the model were selected, and the Random Forest models were run again only with these three miRNAs. Re-sampling of the asthma samples was performed to balance out the weight of each class in the model. The first model was carried out to predict the potential of these miRNAs to distinguish healthy individuals from individuals with asthma for diagnosis, whereas the second model was capable of classifying the subjects as healthy subjects, or into different subgroups of individuals with asthma depending on its severity (intermittent, mild persistent, moderate persistent, or severe persistent).

The ROC curve analysis, the logistic regression model with qualitative predictors, and the Random Forest model with a cross-validation being performed ten times, were performed by means of the R computer program (https://www.r-project.org/).

## Claims

1. An *in vitro* method for selecting subjects with the risk of suffering asthma, which method comprises: (a) measuring the expression pattern or level of at least miR-185-5p obtained from a biological blood, plasma, or serum sample isolated from the subjects to be selected; and (b) comparing said expression pattern or level of at least miR-185-5p of the subjects to be selected with an expression pattern or level in a healthy individual, wherein higher relative expression levels of miR-185 in the subjects to be selected compared with the healthy individuals are indicative of said subject having asthma or having a risk of suffering asthma.

2. The method according to the claim 1, which comprises: (a) measuring the expression pattern or level of at least miR-185-5p and miR-144-5p, and miR-1246 or miR-185-5p, and miR-320a, and miR-320b, obtained from a biological blood, plasma, or serum sample isolated from the subjects to be selected; and (b) comparing said expression pattern or level of any of said combinations of the subjects to be selected with an expression pattern or level in a healthy individual, wherein higher relative expression levels of miR-185-5p and miR-144-5p, and miR-1246 or miR-185-5p, and miR-320a, and miR-320b in the subjects to be selected compared with the healthy individuals are indicative of said subject having asthma or having a risk of suffering asthma.

3. An *in vitro* method for selecting subjects with the risk of suffering mild intermittent or persistent asthma, which method comprises: (a) measuring the expression pattern or level according to any of claims 1 or 2; and (b) comparing said expression pattern or level of the subjects to be selected with an expression pattern or level in a healthy individual, wherein higher relative expression levels of miRNAs identified in step a) in the subjects to be selected compared with the healthy individuals are indicative of the individual suffering mild intermittent or persistent asthma.

4. An *in vitro* method for selecting subjects with the risk of suffering moderate or severe persistent asthma, which method comprises: (a) measuring the expression pattern or level according to any of claims 1 or 2; and (b) comparing said expression pattern or level of the subjects to be selected with an expression pattern or level in a healthy individual, wherein higher relative expression levels of miRNAs identified in step a) in the subjects to be selected compared with the healthy individuals are indicative of the individual suffering moderate or severe persistent asthma.

5. An *in vitro* method for selecting subjects with the risk of suffering moderate or severe persistent asthma or mild intermittent or persistent asthma, which method comprises:
(a) measuring the expression pattern or level according to any of claims 1 or 2; and
(b) comparing said expression pattern or level of the subjects to be selected with an expression pattern or level in a healthy individual, wherein higher relative expression levels of miRNAs identified in step a) in the subjects to be selected compared with the healthy individuals are indicative of the individual suffering moderate or severe persistent asthma or of the individual developing mild intermittent or persistent asthma.

6. An *in vitro* method for selecting subjects with the risk of suffering asthma before age 18, during adolescence, and those with the risk of suffering asthma during adulthood (>age 18), which method comprises: (a) measuring the expression pattern or level of at least miR-185-5p obtained from a biological sample isolated from the subjects to be selected; and (b) comparing said expression pattern or level of at least miR-185-5p of the subjects to be selected with a pre-established expression pattern or level, wherein the difference in the expression of at least miR-185-5p with respect to said pre-established expression pattern or level is indicative of the individual having the risk of developing asthma during adulthood or during adolescence.

7. An *in vitro* method for the diagnosis/prognosis of a subject who is suspected of suffering asthma, which method comprises steps a) and b) of any of the methods of claims 1 to 5, and optionally (c) confirming the presence of asthma and/or the severity of the disease by means of a clinical examination.

8. A method for obtaining useful data for the *in vitro* diagnosis/prognosis of asthma, particularly distinguishing mild intermittent or persistent asthma from moderate or severe persistent asthma, which method comprises steps a) and b) of any of the methods of claims 1 to 5.

9. An *in vitro* method for classifying subjects into one of the following categories: healthy individual, individual with intermittent asthma, mild persistent asthma, moderate persistent asthma, or severe persistent asthma, which method comprises: (a) measuring the expression pattern or level of at least miR-185-5p obtained from a biological sample isolated from the serum, plasma, or blood of the subject to be classified; and (b) comparing said expression pattern or level of at least miR-185-5p of the subject to be classified with a pre-established expression pattern or level for each of said markers, wherein the difference in the expression of at least miR-185-5p with respect to said pre-established expression pattern or level allows classifying said subject with the risk of suffering asthma into any of the following categories: healthy individual, individual with intermittent asthma, mild persistent asthma, moderate persistent asthma, or severe persistent asthma.

10. The *in vitro* method according to the preceding claim, which comprises: (a) measuring the expression pattern or level of at least miR-185-5p in combination with the miRNAs, miR-320a and miR-144-5p, obtained from a biological sample isolated from the serum, plasma, or blood of the subject to be classified; and (b) comparing said expression pattern or level of at least miR-185-5p in combination with the miRNAs, miR-320a and miR-144-5p, of the subject to be classified with a pre-established expression pattern or level for each of said markers, wherein the difference in the expression of at least miR-185-5p in combination with miR-320a and miR-144-5p with respect to said pre-established expression pattern or level allows classifying said subject with the risk of suffering asthma as healthy individual, individual with intermittent asthma, mild persistent asthma, moderate persistent asthma, or severe persistent asthma.

11. An *in vitro* method for monitoring response to a therapy or for monitoring the progression of asthma, particularly distinguishing asthma severity, more particularly distinguishing mild intermittent or persistent asthma from moderate or severe persistent asthma, in a subject suffering said disease, which method comprises steps a) and b) of any of the methods of claims 1 to 10.

12. A method for treating subjects suffering asthma, particularly distinguishing asthma severity, more particularly distinguishing mild intermittent or persistent asthma from moderate or severe persistent asthma, which method comprises steps a) and b) of any of the methods of claims 1 to 10, and (c) treating the patient who has been diagnosed with asthma, particularly distinguishing mild intermittent or persistent asthma from moderate or severe persistent asthma.

13. The method according to any of the preceding claims, wherein the subject is a human subject.

14. Use of a kit comprising reagents which detect biomarkers for determining a differential expression level of at least miR-185-5p, wherein the overexpression or differences in the expression of miR-185-5p is indicative of the risk of having asthma, particularly distinguishing asthma severity, more particularly distinguishing mild intermittent or persistent asthma from moderate or severe persistent asthma, for diagnosing the risk of having asthma *in vitro,* particularly distinguishing mild intermittent or persistent asthma from moderate or severe persistent asthma.

15. Use of the kit according to the preceding claim, wherein said reagents are selected from the group consisting of all or at least one of the following: i) primers specific for the miRNA or miRNA combinations as defined in any of said aspects or preferred embodiments which are capable of producing primer-linked miRNA sequences; ii) reverse transcription means for producing cDNA from the primer-linked miRNA sequences of i); iii) means such as primers capable of amplifying the cDNAs derived from the primer-linked miRNA sequences as defined in i); iv) means for transcribing the amplified cDNAs to produce sense target RNA; and v) a population of antisense miRNA probes capable of detecting the sense target RNA of iv).
